# EUROPÄISCHE PATENTANMELDUNG

(11) **EP 0 857 486 A2**
(43) Veröffentlichungstag der Anmeldung: **12.08.1998**
(21) Anmeldenummer: 98101065.5
(22) Anmeldetag: 22.01.1998
(51) Int. Cl.: A61K 35/74, A61K 39/02

(54) **Gemische äusserer Membranen und/oder Zellwände von Bakterien zur oralen Immunisierung gegen Schleimhautinfektionen**

(30) Priorität: 30.01.1997 DE 19703437
(71) Anmelder: Sankyo Pharma GmbH, 81379 München (DE)
(72) Erfinder: Faller, Anton, Dr., 81249 München (DE); Ellenrieder, Max, Dr., 85579 Neubiberg (DE); Hofbauer, Christina, Dr., 85737 Ismaning (DE); Wick, Georg, Prof. Dr., 6080 Igls (AT); Wolf, Hugo, Dr., 6073 Sistrans (AT)
(74) Vertreter: Zumstein, Fritz, Dr.

(57) **Zusammenfassung**

Gegenstand der Erfindung ist ein Gemisch enthaltend äußere Membranen gramnegativer Bakterien und / oder Zellwände grampositiver Bakterien, die jeweils Schleimhautinfektionen auslösen, im wesentlichen frei von anderen Zellbestandteilen sowie dessen Herstellung und ein Arzneimittel.

## Beschreibung

Vakzinierung beruht im wesentlichen auf der Aktivierung einer schützenden Immunantwort gegen einen Krankheitserreger durch ein immunogenes Antigen. Die Immunantwort ist im allgemeinen spezifisch für das bei der Vakzinierung verabreichte Antigen. Sie ist durch die Bildung zellständiger oder frei zirkulierender, gegen das Antigen spezifisch gerichteter Antikörper bedingt. Daneben gibt es aber auch eine unspezifische Stimulierung des Immunsystems, wobei beispielsweise die Phagocytose aktiviert oder die Proliferation aller Lymphozyten angeregt wird. Die spezifische Immunantwort kann sowohl systemisch durch zirkulierende Antikörper als auch lokal in Schleimhäuten erfolgen. Bislang wird die Immunisierung vorwiegend mit Organismen, z.B. Bakterien oder deren Lysaten, auf parenteralem Weg vorgenommen. Daneben wird in einigen Fällen auch durch orale Verabreichung von Antigenen immunisiert.
Bei der perenteralen Immunisierung wird vorwiegend eine systemische Immunantwort erzeugt. Bei der oralen Immunisierung mit Bakterien oder Lysaten von Bakterien kommt es zur lokalen Aktivierung von Abwehrmechanismen in den Schleimhäuten, nämlich zur Aktivierung des Mucosa Associated Lymphoid Tissue (MALT) von Mensch und Tier. Die Schleimhautoberflächen sind Eintrittspforte für Infektionserreger. Deshalb ist die Schleimhautimmunität wichtig als 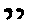 first line of defense". Das Schleimhaut assoziierte Immunsystem schützt den Körper vor dem Eindringen von Mikroben in tiefere Regionen.

Fur die perenterale Immunisierung mit Keimen werden hitzeabgetötete ganze Bakterien, wie z.B. Bordetella pertussis (ROBINSON A. et al: Pertussis vaccine: present status and future prospects. Vaccine 1985; 3: 11 - 22 und EP 0173241), Bakterienlysate (EP 0139551) oder Bestandteile von Bakterien, insbesondere Bestandteile der Kapsel bestimmter kapselbildender Bakterien, zum Beispiel Polyoside (EP 0071515) verwendet. Hierbei wird eine systemische Immunantwort erzeugt.
Erste Erfahrungen mit oraler Immunisierung basieren auf einem epidemiologischen Experiment im Jahre 1948. Hier wurden in einem typhusverseuchten Gebiet Personen mit attenuierten ganzen Typhuskeimen oral geimpft (RAETTIG H: Erfahrungen und vergleichende Betrachtungen über die Erfolge der parenteralen und peroralen Typhusschutzimpfung. Z. Immunitätsforschung 1950; 108: 165). Es ist hierbei nicht beschrieben, ob es sich um eine lokale oder systemische Immunantwort handelte.

Desweiteren werden auch Bakterienlysate oral verabreicht. Eine Möglichkeit der Lyse von Bakterien besteht in einem alkalischen Aufschluß (EP 0269928, DE 4104728 und DE OS 2917730). Eine weitere Möglichkeit Bakterien zu lysieren, bietet der physikalische Aufschluß, z.B. können durch mechanische Reibung die Zellwände abgebrochen werden. Solche Bakterienlysate sind Gemische sämtlicher Zellkomponenten. Diese Lysate waren in der Lage nach oraler Gabe eine lokale Immunanwort in Form einer erhöhten Produktion von antigenspezifischem IgA durch Lungenzellen zu induzieren (RUEDL CH et al: Immune response in the lungs following oral immunization with bacterial lysate of respiratory pathogens. Clin. Diagn. Lab. Immunol. 1994; 1: 150 - 154).

Weiterer zu berücksichtigender Stand der Technik ergibt sich aus folgenden Dokumenten. DE-C-42 31 543 beschreibt lösliche Fraktionen aus Zellwänden von Eubakterien, welche mittels Proteinase-Abbau erhalten werden und entzündungshemmende Aktivität aufweisen. Die Fraktionen werden zur Behandlung von Hautentzündungen verwendet. DE-A-27 35 411 offenbart Mischungen von bakterieller RNA mit Membran-Saccharidfraktionen bestimmter gramnegativer Bakterien, wie Klebsiella. Solche Präparate werden als Vaccine zur parenteralen Verabreichung verwendet, um bronchiopulmonalen Infektionen vorzubeugen. FR-A-2 475 900 beschreibt Vaccinepräparate, enthaltend Komplexe bakterieller ribosomaler RNA mit verschiedenen anderen Zell-Fraktionen für die parenterale Verabreichung. EP-A-0 416 892 beschreibt Zell-Homogenate oder -Fraktionen, erhalten durch enzymatischen Abbau aus bestimmten Bakterien, zur Behandlung von Diarrhö. Bessler und Sedelmeier (Arzneim. Forsch. 43, 502-507, 1993) beschreiben die Wirkungen von Broncho-Vaxom® und Uro-Vaxom® auf die Bildung spezifischer Antikörper in Mäusen nach parenteraler Verabreichung.

Daneben wurde auch die unspezifische Immunstimulation durch bestimmte Bestandteile oder Fraktionen von Bakterien beschrieben, wie z.B. Glycoproteine (DE OS 3 029 111, DD 289 200), Galaktanextrakte (EP 0408 444) und Proteoglykane (EP 0 089 266).

Besonders vorteilhaft im Sinne eines umfassenden Infektionsschutzes ist neben der unspezifischen Aktiverung die Erzeugung sowohl einer systemischen als auch lokalen spezifischen Immunantwort. Die Erzeugung einer spezifischen systemischen und zugleich einer spezifischen lokalen Immunantwort nach oraler Verabreichung von Bakterien oder deren Kompenenten ist bisher nicht bekannt geworden.

Aufgabe der Erfindung war es, zum Schutz vor Schleimhautinfektionen ein oral wirksames Mittel zur Verfügung zu stellen, das eine spezifische systemische und lokale Immunantwort induziert.

Die Lösung der Aufgabe wurde in einem Gemisch von äußeren Membranen gramnegativer Bakterien und / oder Zellwänden grampositiver Bakterien gefunden, die Schleimhautinfektionen auslösen können.

Gegenstand der Erfindung ist daher ein Gemisch enthaltend äußere Membranen gramnegativer Bakterien, die Schleimhautinfektionen auslösen können.

Gegenstand der Erfindung ist ferner ein Gemisch enthaltend Zellwände grampositiver Bakterien, die Schleimhautinfektionen auslösen können.

Gegenstand der Erfindung ist auch ein Gemisch enthaltend äußere Membranen gramnegativer Bakterien und Zellwände grampositiver Bakterien, die Schleimhautinfektionen auslösen können.

Die erfindungsgemäßen Gemische enthalten Bestandteile von mindestens zwei, insbesondere von zwei bis 10 verschiedenen gramnegativen und / oder grampositiven Bakterien. Bevorzugt sind Gemische, die Bestandteile von drei bis fünf verschiedenen Bakterien enthalten. Eine besonders bevorzugte Ausführungsform enthält dabei Bestandteile sowohl von gramnegativen als auch von grampositiven Bakterien.

Die Bakterien, deren Bestandteile das erfindungsgemäße Gemisch enthalt, sind aus folgenden Genera Schleimhautinfektionen auslösender Bakterien ausgewählt: Klebsiella, Escherichia, Proteus, Gardnerella, Haemophilus, Branhamella, Veillonella, Capnocytophaga, Rothia, und Streptococcus.

Die Schleimhautinfektionen, die mit erfindungsgemäßen Gemischen behandelt werden können, betreffen die Atemwege, die Mundhölhe, die Nebenhöhlen, das Mittelohr und den Urogenitaltrakt. Für die einzelnen Zielorgane sind unterschiedliche Bakterien als Infektionsquelle relevant. Die erfindungsgemäßen Gemische können daher je nach Zielorgan für den Infektionsschutz Bestandteile unterschiedlicher Bakterien enthalten, vorzugsweise Bestandteile für das jeweilige Organ relevanter Infektionskeime.

Für Atemwegsinfektionen sind folgende Erreger relevant: Klebsiella pneumoniae, Haemophilus influenzae, Branhamella catarrhalis und Streptococcus pneumoniae. Im Falle von Infektionen der Nebenhöhlen (Sinusitis) sind folgende Erreger von Bedeutung: Haemophilus influenzae, Branhamella catarrhalis und Streptococcus pneumoniae. Entzündungen des Mittelohres (Otitis media) werden besonders häufig von Branhamella catarrhalis, Streptococcus pneumoniae oder Haemophilus influenzae ausgelöst. Für Infektionen der Mundhöhle (Karies, Parodontitis) sind folgende Bakterien von Bedeutung: Streptococcus mutans, Veillonella sp., Streptococcus salivarius, Capnocytophaga sp. und Rothia dentocariosa. Im Falle einer Entzündung des Urogenitaltraktes sind Escherichia coli, Klebsiella pneumoniae, Proteus sp. und Gardnerella vaginalis von besonderer Bedeutung.

Für die Behandlung zur Prophylaxe vor Infektionen des Respirationstrakts eignet sich besonders ein Gemisch enthaltend äußere Membranen von Klebsiella pneumoniae, Haemophilus influenzae, Branhamella catarrhalis und Zellwände von Streptococcus pneumoniae.

Die äußeren Membranen bzw. die Zellwände können von jeder Art der betreffenden Bakterien stammen. Vorzugsweise verwendet man Bestandteile handelsüblicher Bakterienstämme, wie sie beispielsweise von der American Type Culture Collection erhältlich sind.

Die Mengenanteile der einzelnen Bestandteile (äußere Membranen und / oder Zellwände der verschiedenen Bakterien) können gleich oder verschieden sein und jeweils von 5 bis 70 Gewichtsprozent betragen, bezogen auf das Gemisch. Bevorzugt sind Gemische, in denen die Mengenanteile der Bestandteile der einzelnen Bakterien gleich sind. Je nach Immunogenität der verwendeten Bakterien können die Gewichtsanteile der Bestandteile auch höher oder niedriger sein.

Die erfindungsgemäßen Gemische sind im wesentlichen frei von anderen Zellbestandteilen. 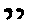 Im wesentlichen frei" bedeutet, daß die Gemische höchstens 10 Prozent an anderen Zellbestandteilen, insbesondere zwischen 1 und 5 Prozent enthalten.

Gegenstand der Erfindung ist ferner ein Verfahren zur Herstellung des erfindungsgemäßen Gemisches der äußeren Membranen und / oder der Zellwände der Bakterien, das dadurch gekennzeichnet ist, daß man die äußeren Membranen gramnegativer Bakterien durch Ablösen der Membran vom Sphäroplasten und anschließende Abtrennung der Membran vom Sphäroplasten gewinnt und / oder daß man die Zellwände grampositiver Bakterien durch mechanischen Aufschluß der Bakterien und Abtrennung der Zellwände von den restlichen Zellbestandteilen durch Zentrifugation gewinnt, daß man sie reinigt und daß man die gereinigten äußeren Membranen gramnegativer Bakterien und / oder die gereinigten Zellwände grampositiver Bakterien miteinander mischt. Vor der Gewinnung der äußeren Membranen und / oder der Zellwände der Bakterien züchtet man die Mikroorganismen in üblicher Weise in wässrigen Nährmedien, die Peptone, Hefeextrakt, Zucker, Salze und spezielle Wachstumsfaktoren enthalten. Die dabei gewonnenen Biomassen werden durch Hitze (60°C - 70°C) inaktiviert und durch Zentrifugation oder Ultrafiltration abgetrennt.

Zur Gewinnung der äußeren Membranen von gramnegativen Bakterien werden zum Beispiel deren Zellwände in üblicher Weise in einem ersten Schritt enzymatisch aufgelöst. Als Folge entstehen Sphäroplasten mit locker anhaftenden äußeren Membranen, die durch Ultrafiltration eingeengt werden. Ein anschließendes Aufquellen des Retentates, zum Beispiel in einem hypotonischen Puffer, verursacht das Ablösen der äußeren Membranen von den Sphäroplasten.

Besonders hervorzuheben ist die Tatsache, daß die Trennung der äußeren Membranen von den Sphäroplasten mit Hilfe einer Membranfiltration möglich ist. Vorzugsweise bedient man sich in diesem Falle dabei der Cross-flow-Filtrationstechnik, insbesondere unter Verwendung von Filtermaterial aus Nylon mit einer Porengröße von 0,45 µm.

Die Reinigung der abgetrennten äußeren Membranen wird durch Waschen mit entsalztem Wasser in einem gesonderten Ultrafiltrationsschritt erreicht.

Zur Gewinnung der Zellwände von grampositiven Bakterien werden beispielsweise disintegrierte Zellen in üblicher Weise zentrifugiert, um die löslichen Komponenten und Zellbruchstücke voneinander zu trennen Nach Resuspension der Zellwände in Phosphatpuffer werden in einem weiteren Zentrifugationsschritt bei niedriger g-Zahl als Reinigungschritt die noch vorhandenen Membranteile entfernt.

Vorzugsweise werden die isolierten äußeren Membranen sowie die Zellwände bei -20°C gelagert.

Die erfindungsgemäßen Gemische werden beispielsweise dadurch hergestellt, daß man die isolierten, tiefgefrorenen äußeren Membranen von gramnegativen Bakterien und / oder die isolierten, tiefgefrorenen Zellwände von grampositiven Bakterien auftaut, nach vorgegebenen Trockengewichtsanteilen mischt, in Wasser oder in einer wässrigen Trägersubstanzlösung gleichmäßig suspendiert und lyophilisiert oder sprühtrocknet.

Als Trägersubstanzen werden die üblichen wasserlöslichen Stoffe, beispielsweise ausgewählt aus der Gruppe der Zucker, insbesondere Mannitol, verwendet.

Die äußeren Membranen und Zellwände zeichnen sich dadurch aus, daß sie partikulärer Struktur und nicht wasserlöslich sind. Ihre charakteristischen Protein- bzw. Lipopolysaccharid (LPS)-Strukturen lassen sich mit Hilfe der SDS-Gelelektrophorese darstellen.

Im Zusassmenhang mit der vorliegenden Erfindung wurde überraschend gefunden, daß Gemische äußerer Membranen und / oder Zellwände verschiedener Bakterien anders als Bakterienlysate sowohl eine spezifische lokale als auch eine spezifische systemische Immunantwort nach oraler Gabe induzieren. Die äußeren Membranen und die Zellwände steigern nach oraler Applikation die Abwehrmechanismen in der Lunge, indem Lymphozyten aus der Lunge von immunisierten Tieren in der Lage sind, in hohem Maße antigenspezifisches IgA zu produzieren. Daneben induzieren äußere Membranen und Zellwände bei oral immunisierten Tieren im Serum erhöhte antigerspezifische IgG-Titer, verglichen zu unbebandelten Tieren.
Deshalb eignen sich die erfindungsgemäßen Gemische zur Prophylaxe von Infektionen der Schleimhäute, bevorzugterweise von Infektionen der Atemwege. Die schützenden Wirkungen der erfindungsgemäßen Gemische wurden in den nachstehend beschriebenen Versuchen gezeigt.

### Versuch 1:

### Einfluß auf die lokale spezifische Infektabwehr

Am Beispiel der äußeren Membran von Branhamella catarrhalis wird der Einfluß auf das lokale spezifische Immunsystem geprüft. Weibliche Balb/c Mäuse wurden mit 500 µg /Tier an den Tagen 1, 2, 3, 4, 5 und 15, 16, 17, 18, 19 peroral immunisiert. Am Tag 29 wurden die Tiere getötet, die Lungen sowie die Genitaltrakte entnommen und Zellpopulationen isoliert. In den Zellkulturüberständen dieser Zellpopulationen wurde antigenspezifisches IgA mit Hilfe eines time-resolved fluoroimmunoassays, der bei RUEDL et al., J. Immunol. Methods 168 (1994), 61 - 67 beschrieben ist, in signifikant größeren Mengen im Vergleich zu Kulturöberständen von Zellen unbehandelter Tiere gefunden (Abb.1)

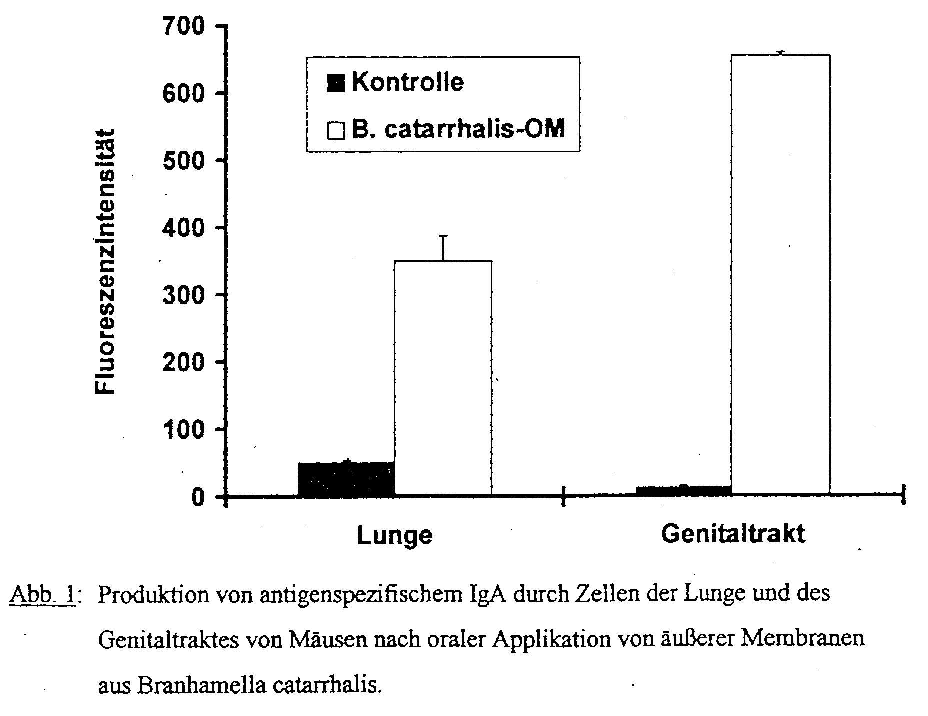

Verwendet man anstelle der äußeren Membran von Branhamella catarrhalis zur Immunisierung das Gemisch aus Beispiel 3, so erhält man im Lungengewebe eine Steigerung des Branhamella-spezifischen IgA um das sechsfache und im Genitaltrakt um das achtfache gegenüber unbehandelten Kontrollen.

### Versuch 2:

### Einfluß auf die systemische spezifische Infektabwehr

Von den Tieren aus Versuch 1 wurde auch Blut entnommen und im Serum auf antigenspezifisches IgG geprüft. Abbildung 2 zeigt, daß die orale Immunisierung mit äußeren Membranen von Branhamella catarrhalis erhöhte antigenspezifische IgG-Spiegel induziert.

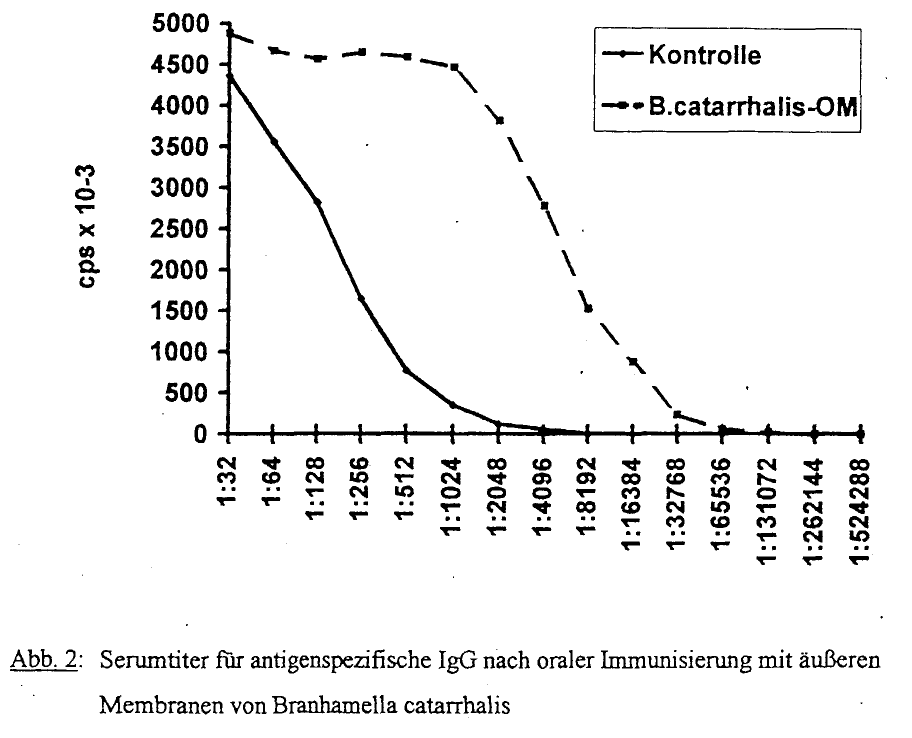

Diese Serumantikörper sind spezifisch gegen die äußeren Membran-Antigene von Branhamella catarrhalis gerichtet. Die Erhöhung des Titers beträgt ca. den Faktor 32. Bei Verwendung eines Gemisches gemäß Beispiel 3 beträgt die Titererhöhung den Faktor 16.

### Versuch 3:

### Infektionsschutz durch ein Gemisch aus äußeren Membranen und Zellwänden

Ein Gemisch gemäß Beispiel 3 wurde peroral Mäusen verabreicht an den Tagen 1, 2, 3, 4, 5 und 15, 16, 17, 18, 19.
Am Tag 29 wurden die Tiere intranasal mit einem mäusepathogenen Stamm von Klebsiella pneumoniae bzw. Streptococcus pneumoniae infiziert und die Überlebensraten festgestellt (Abb. 3 und 4).

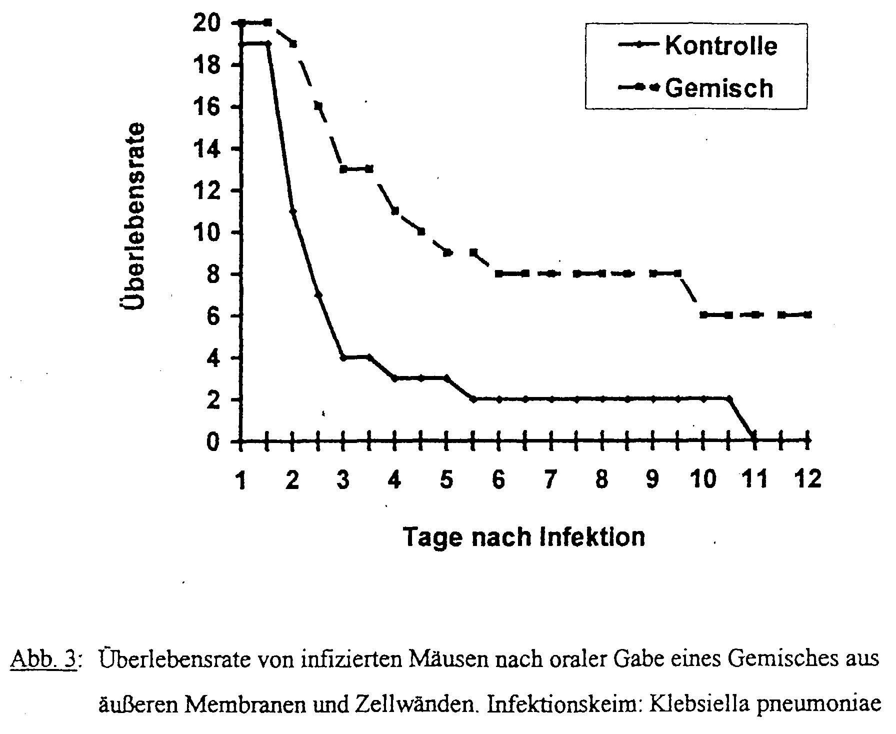

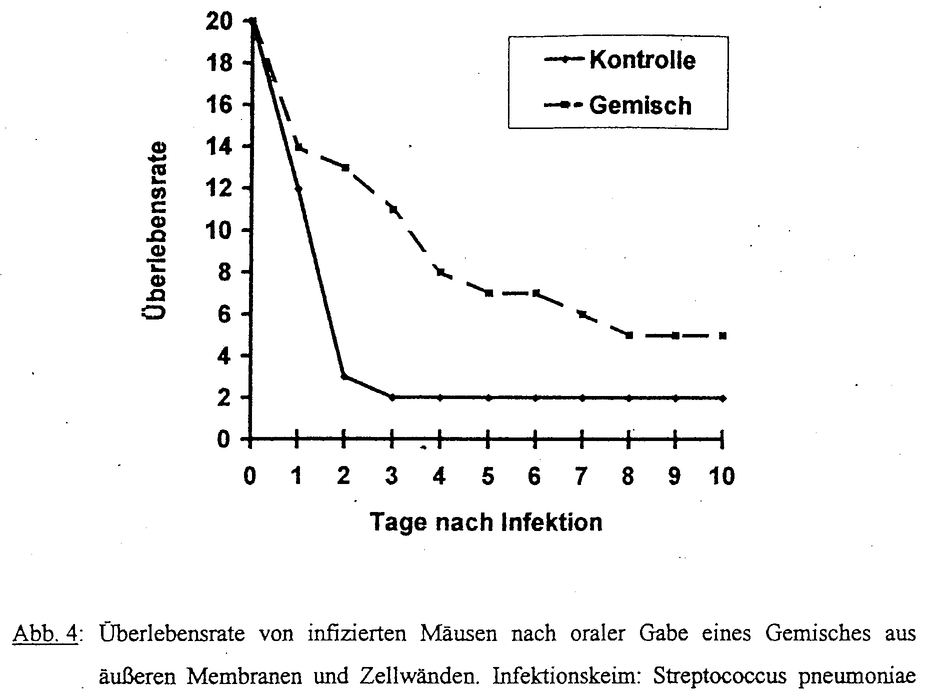

Die Ergebnisse aus beiden Infektionsexperimenten zeigen einen signifikant besseren Infektionsschutz für Tiere, die mit dem Gemisch aus äußeren Membranen und Zellwand oral behandelt wurden, verglichen mit unbehandelten Tieren.

Gegenstand der Erfindung ist ferner ein Arzneimittel für Tier und Mensch bestehend aus oder enthaltend ein erfindungsgemäßes Gemisch aus äußeren Membranen gramnegativer Bakterien und / oder Zellwänden grampositiver Bakterien, die Schleimhautinfektionen auslösen.

Das Gemisch der Bakterienbestandteile kann alleine oder zusammen mit üblichen Träger- und Hilfsstoffen im Arzneimittel enthalten sein. Das Arzneimittel wird vorzugsweise oral verabreicht.
Als Verabreichungsformen zur oralen Applikation kommen Kapseln, Tabletten, Lutschtabletten, Kaugummi, Granulat, Saft oder Tropfen in Frage.

Tabletten, Dragees, Kapseln, Pillen und Granulate können die erfindungsgemäßen Gemische neben den üblichen Trägerstoffen enthalten, wie a) Füll- und Streckmittel, zum Beispiel Stärken, Milchzucker, Rohrzucker, Glucose, Mannitol und Kieselsäure, b) Bindemittel, zum Beispiel Carboxymethylcellulose, Alginate, Gelatine, Polyvinylpyrrolidon, c) Feuchthaltemittel, zum Beispiel Glycerin, d) Sprengmittel, zum Beispiel Agar-Agar, Calciumcarbonat und Natrium-bicarbonat, e) Lösungsverzögerer, zum Beispiel Paraffin und f) Resorptionsbeschleuniger, g) Netzmittel, zum Beispiel Cetylalkohol, Glycerinmonostearat, h) Adsorptionsmittel, zum Beispiel Kaolin und Bentonit und i) Gleitmittel, zum Beispiel Talkum, Calcium- und Magnesimstearat und feste Polyethylenglycole oder Gemische der unter a) bis i) aufgeführten Stoffe.

Die Tabletten, Dragees, Kapseln, Pillen und Granulate können mit den üblichen gegebenenfalls Opakisierungsmittel enthaltenden Überzügen, zum Beispiel Zucker, Überzugslacke, versehen sein und auch so zusammengesetzt sein, daß sie die erfindungsgemäßen Gemische nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Tagesdosis des Gemisches liegt zwischen 0,05 mg und 5 mg, vorzugsweise zwischen 0,1 mg und 2 mg, wobei der Anteil an Bestandteilen (äußere Membran bzw. Zellwand) eines einzelnen Keims zwischen 0,0025 mg und 3,5 mg, bevorzugt zwischen 0,005 mg und 1,4 mg liegt.

Die nachstehenden Beispiele dienen der näheren Erläuterung der Erfindung ohne sie darauf zu beschränken.

### Beispiel 1:

### Züchtung und Gewinnung der Biomasse der angegebenen Stämme

**a) Klebsiella pneumoniae**
   Der Stamm ATCC 13 883 wird vorzugsweise im Fermenter bebrütet, wobei das Grundmedium je Liter Wasser 16 g Hefeextrakt, 20 g Glucose, 1 g NaCl, 10 g Na₂HPO₄ und 3g KH₂PO₄ enthält, wobei diese Werte um ± 5 % schwanken können. Die Bebrütung erfolgt bei 37°C und Bewegung, bei einem konstanten pH-Wert von 6,8 solange bis der Glucose-Anteil aufgebraucht ist. Die gewachsenen Kulturen werden durch Aufheizen auf 60°C - 70°C über maximal 1 Stunde inaktiviert.
**b) Haemophilus influenzae**
   Der Stamm ATCC 19 418 wird vorzugsweise im Fermenter bebrütet, wobei das Grundmedium je Liter Wasser 10 g Pepton aus Sojabohnenmehl, 5 g Hefeextrakt, 5 g Glucose, 1g NaCl, 1 mg Hämin (vom Pferd) und 0,5 mg NAD enthält, wobei die Werte um ± 5 % schwanken können.
   Die Bebrütung erfolgt bei 37°C und einem pH-Wert von 7,4 unter Belüftung und Bewegung, bis die Trübungsmeßsonde keine Zunahme an Biomasse anzeigt. Die gewachsenen Kulturen werden durch Aufheizen auf 60°C - 70°C über maximal 1 Stunde inaktiviert.
**c) Branhamella catarrhalis**
   Der Stamm ATCC 25 240 wird vorzugsweise im Fermenter bebrütet, wobei das Grundmedium je Liter Wasser 5 g Pepton aus Sojabohnenmehl, 7 g Hefeextrakt, 5 g NaCl und 1,5 g KH₂PO₄ enthält, wobei diese Werte um ± 5 % schwanken können. Die Bebrütung erfolgt bei 37°C und einem pH-Wert von 7,4 unter Belüftung und Bewegung während 10 - 14 Stunden. Die gewachsenen Kulturen werden durch Aufheizen auf 60°C - 70°C über maximal 1 Stunde inaktiviert.
**d) Streptococcus pneumoniae**
   Der Stamm ATCC 6 303 wird vorzugsweise im Fermenter bebrütet, wobei das Grund-medium je Liter Wasser 15 g Pepton aus Sojabohnenmehl, 10 g Hefeextrakt, 5 g Glucose, 2 g NaCl, 0,4 g Na₂HPO₄ und 1 mg Hämin (vom Pferd) enthält, wobei die Werte um ± 5 % schwanken können.
   Die Bebrütung erfolgt bei 37°C und Bewegung, bei einem konstanten pH-Wert von 5,8. Zur Steigerung der Biomasseausbeute kann in dieser Wachstumsphase Glucose zusätzlich zugegeben werden. Die gewachsenen Kulturen werden durch Aufheizen auf 60°C - 70°C über maximal 1 Stunde inaktiviert.

Nach der thermischen Inaktivierung der Kulturen wird ihre Zellzahl mikroskopisch ermittelt. Die Biomasse wird vom Medium absepariert, in entsalztem Wasser oder physiologischer NaCl-Lösung gewaschen und bis zur Weiterverarbeitung als Suspension mit 6- 10 % TG-Anteil eingefroren.

### Beispiel 2:

### Extraktion und Reinigung der äußeren Membran oder Zellwand der angegebenen Stämme

**a) Klebsiella pneumoniae**
   Die inaktivierten ganzen Zellen des Stammes gemäß Beispiel 1a) werden in 10 mM TRIS/HCl-Puffer (pH 7,8), der 0,5 M Saccharose enthält, suspendiert und soweit verdünnt, daß die resultierende Suspension einen OD-Wert von 80 besitzt. Man gibt der Suspension 600 mg/l Lysozym zu und schüttelt bei 30°C für 7 Stunden. Nach der Inkubationszeit wird die doppelte Ansatzmenge einer 1,5 mM EDTA-Lösung (pH 7,6) zugegeben und für 16 - 18 Stunden kühl gelagert.
   Man engt den Ansatz mittels Ultrafiltration ein und suspendiert in 10 mM TRIS/HCl-Puffer (pH 7,2).
   Man trennt die Membranen mittels Ultrafiltration ab, wobei eine Filtermembran mit 0,45 µm Porengröße Verwendung findet.
   Man engt die im Filtrat sich befindenden äußeren Membranen durch Ultrafiltration ein, wäscht 2 mal in entsalztem Wasser, bestimmt den Trockengewichsanteil und lagert die so gewonnenen äußeren Membranen bei -20°C.
**b) Haemophilus influenzae**
   Die inaktivierten ganzen Zellen des Stammes gemäß Beispiel 1b) werden in 10 mM TRIS/HCl-Puffer (pH 7,8), der 0,5 M Saccharose enthält, suspendiert und soweit verdünnt, daß die resultierende Suspension einen OD-Wert von 40 besitzt. Man gibt der Suspension EDTA bis zu einer Endkonzentration von 5 mM zu und inkubiert 15 Minuten. Man gibt dem Ansatz Lysozym in einer Konzentration von 0,1 g/l zu und inkubiert 4 Stunden.
   Man trennt die Membranen mittels Ultrafiltration ab, wobei eine Filtermembran mit 0,45 µm Porengröße Verwendung findet.
   Man engt die im Filtrat sich befindenden äußeren Membranen durch Ultrafiltration ein, wäscht 2 mal in entsalztem Wasser, bestimmt den Trockengewichtsanteil und lagert die so gewonnenen äußeren Membranen bei -20°C.
**c) Branhamella catarrhalis**
   Die inaktivierten ganzen Zellen des Stammes gemäß Beispiel 1c) werden in 10 mM TRIS/HCl-Puffer (pH 7,8), der 0,5 M Saccharose enthält, suspendiert und soweit verdünnt, daß die resultierende Suspension einen OD-Wert von 80 besitzt. Man gibt der Suspension 200 mg/l Lysozym zu und schüttelt bei 30°C für 7 Stunden. Nach der Inkubationszeit wird die doppelte Ansatzmenge einer 1,5 mM EDTA-Lösung (pH 7,6) zugegeben und für 16 - 18 Stunden kühl gelagert.
   Man engt den Ansatz mittels Ultrafiltration ein und suspendiert in 10 mM TRIS/HCl-Puffer (pH 7,2).
   Man trennt die Membranen mittels Ultrafiltration ab, wobei eine Filtermembran mit 0,45 µm Porengröße Verwendung findet.
   Man engt die im Filtrat sich befindenden äußeren Membranen durch Ultrafiltration ein, wäscht 2 mal in entsalztem Wasser, bestimmt den Trockengewichtsanteil und lagert die so gewonnenen äußeren Membranen bei -20°C.
**d) Streptococcus pneumoniae**
   Die inaktivierten ganzen Zellen des Stammes gemäß Beispiel 1d) werden nach dem Auftauen mit entsalztem Wasser zu einer Suspension mit einem Trockengewichtsanteil von 6 % verdünnt, wobei dieser Wert um ± 1 % schwanken kann.
   Man schließt diese Zellen mechanisch mit Glasperlen in einer Zellmühle auf, was mikroskopisch kontrolliert wird.
   Man entfernt die löslichen Komponenten mittels Zentrifugation bei 48000 g bei 4°C für 20 Minuten.
   Man suspendiert das Pellet in 0,05 m PBS-Puffer (pH 7,8) bis zum Ausgangsvolumen.
   Man zentrifugiert bei 6000 g für 20 Minuten, 4°C zum Entfernen der Membranteile.
   Man suspendiert das Pellet in 0,05 m PBS-Puffer (pH 7,8) bis zum Ausgangsvolumen.
   Man zentrifugiert bei 48000 g für 20 Minuten, 4°C.
   Man suspendiert das Pellet in einigen Milliliter entsalztem Wasser, bestimmt den Trockengewichtsanteil und lagert die so gewonnenen Zellwände bei -20°C.

### Beispiel 3:

### Gemisch aus äußeren Membranen und Zellwänden

Die tiefgefrorenen Suspensionen der äußeren Membranen gramnegativer Bakterien und Zellwände grampositiver Bakterien werden aufgetaut und zu gleichen Gewichtsteilen (jeweils 20 g) in 10 %iger Mannitollösung (1kg/10.000ml) suspendiert und sprühgetrocknet. Die dafür erforderlichen Suspensionsvolumina sind für Klebsiella pneumoniae 606,0 ml, für Haemophilus influenzae 1020,4ml, für Branhamella catarrhalis 813,0ml und für Streptococcus pneumoniae 636,9ml. Nach dem Hinzufügen von 1kg Mannitol wird mit entsalztem Wasser auf 10.000ml aufgefüllt.

Dabei resultiert ein Gemisch aus

| | |
|---|---|
| Klebsiella pneumoniae | 500 µg |
| Haemophilus influenzae | 500 µg |
| Branhamella catarrhalis | 500 µg |
| Streptococcus pneumoniae | 500 µg |

und 25 mg Mannitol.

### Beispiel 4:

### Gemisch aus äußeren Membranen

Die tiefgefrorenen Suspensionen der äußeren Membranen grammnegativer Bakterien werden aufgetaut und zu gleichen Gewichtsteilen (jeweils 20g) in 10 %iger Mannitollösung (1kg/10.000ml) suspendiert und sprühgetrocknet. Die dafür erforderlichen Suspensionsvolumina sind für Klebsiella pneumoniae 606,0ml, für Haemophilus influenzae 1020,4ml, für Branhamella catarrhalis 813,0ml. Nach dem Hinzufügen von 1kg Mannitol wird mit entsalztem Wasser auf 10.000ml aufgefüllt.

Dabei resultiert ein Gemisch aus

| | |
|---|---|
| Klebsiella pneumoniae | 500 µg |
| Haemophilus influenzae | 500 µg |
| Branhamella catarrhalis | 500 µg |

und 25 mg Mannitol.

### Beispiel 5:

### Gemisch aus Zellwänden

Die tiefgefrorenen Suspensionen der Zellwände grampositiver Bakterien werden aufgetaut und zu gleichen Gewichtsteilen (jeweils 20g) in 10 %iger Mannitollösung (1kg/10.000ml) suspendiert und sprühgetrocknet. Die dafür erforderlichen Suspensionsvolumina sind für Streptococcus pneumoniae 636,9ml, für Streptococcus mutans 675,7ml und für Strepococcus salivarius 800,0ml. Nach dem Hinzufügen von 1kg Mannitol wird mit entsalztem Wasser auf 10.000ml aufgefüllt.

Dabei resultiert ein Gemisch aus

| | |
|---|---|
| Streptococcus pneumoniae | 500 µg |
| Streptococcus mutans | 500 µg |
| Streptococcus salivarius | 500 µg |

und 25 mg Mannitol.

### Beispiel 6:

### Gemisch aus äußeren Membranen

Die tiefgefrorenen Suspensionen der äußeren Membranen gramnegativer Bakterien werden aufgetaut und im angegebenen Gewichtsverhältnis in 20 %iger Mannitollösung (2kg/10.000ml) suspendiert und sprühgetrocknet. Die dafür erforderlichen Suspensionsvolumina sind für Escherichia coli 1265ml (40g), für Proteus vulgaris 649,4ml (20g), für Proteus mirabilis 671,1ml (20g), für Klebsiella oxytoca 1234,6ml (40g) und für Gardnerella vaginalis 1481,5ml (40g). Nach dem Hinzufügen von 2kg Mannitol wird mit entsalztem Wasser auf 10.000ml aufgefüllt.

Dabei resultiert ein Gemisch aus

| | |
|---|---|
| Escherichia coli | 1000 µg |
| Proteus vulgaris | 500 µg |
| Proteus mirabilis | 500 µg |
| Klebsiella oxytoca | 1000 µg |
| Gardnerella vaginalis | 1000 µg |

und 50 mg Mannitol.

### Beispiel 7:

### Gemisch aus äußeren Membranen und Zellwänden

Die tiefgefrorenen Suspensionen der äußeren Membranen gramnegativer Bakterien und Zellwänden grampositiver Bakterien werden aufgetaut und im angegebenen Gewichtsverhältnis in 20 %iger Mannitollösung (2kg/10.000ml) suspendiert und sprühgetrocknet. Die dafür erforderlichen Suspensionsvolumina sind für Haemophilus influenzae 204,1ml (4g), für Branhamella catarrhalis 406,5ml (10g), für Streptococcus mutans 675,7ml. (20g), für Strepococcus salivarius 800,0ml (20g) und für Veillonella parvula 1123,6ml (20g). Nach dem Hinzufügen von 2kg Mannitol wird mit entsalztem Wasser auf 10.000ml aufgefüllt.

Dabei resultiert ein Gemisch aus

| | |
|---|---|
| Haemophilus influenzae | 100 µg |
| Branhamella catarrhalis | 250 µg |
| Streptococcus mutans | 500 µg |
| Streptococcus salivarius | 500 µg |
| Veillonella parvula | 500 µg |

und 50 mg Mannitol.

### Beispiel 8:

### Gemisch aus äußeren Membranen und Zellwänden

Die tiefgefrorenen Suspensionen der äußeren Membranen gramnegativer Bakterien und Zellwände grampositiver Bakterien werden aufgetaut und zu gleichen Gewichtsteilen (jeweils 20g) in 10.000ml entsalztem Wasser suspendiert und lyophilisiert. Die dafür erforderlichen Suspensionsvolumina sind für Klebsiella pneumoniae 606,0ml, für Haemophilus influenzae 1020,4ml, für Branhamella catarrhalis 813,0ml und für Streptococcus pneumoniae 636,9ml.

Dabei resultiert ein Gemisch aus

| | |
|---|---|
| Klebsiella pneumoniae | 500 µg |
| Haemophilus influenzae | 500 µg |
| Branhamella catarrhalis | 500 µg |
| Streptococcus pneumoniae | 500 µg |

### Beispiel 9:

### Arzneimittel

Arzneimittel als Tablette, wobei 130 mg enthalten als Wirkstoffgemisch

| | | |
|---|---|---|
| äußere Membranen von | Klebsiella pneumoniae | 800 µg |
| | Haemophilus influenzae | 400 µg |
| | Branhamella catarrhalis | 1000 µg |
| sowie Zellwände von | Streptococcus pneumoniae | 1000 µg |
| und als Hilfsstoffe | Mannitol | 73,0 mg |
| | Mikrokristalline Cellulose | 51,0 mg |
| | Magnesiumstearat | 2,8 mg |

## Patentansprüche

1. Gemisch enthaltend äußere Membranen gramnegativer Bakterien und / oder Zellwände grampositiver Bakterien, die jeweils Schleimhautinfektionen auslösen, im wesentlichen frei von anderen Zellbestandteilen.

2. Gemisch gemäß Anspruch 1 dadurch gekennzeichnet, daß es die Bestandteile solcher Bakterien enthält, die Infektionen der Atemwege, der Mundhöhle, der Nebenhöhlen, des Mittelohrs und des Urogenitaltraktes auslösen.

3. Gemisch gemäß einem der vorstehenden Ansprüche dadurch gekennzeichnet, daß es die Bestandteile von Bakterien enthält, die aus folgenden Gattungen gramnegativer Bakterien: Klebsiella, Escherichia, Haemophilus, Branhamella, Proteus, Gardnerella, Capnocytophaga, Veillonella und / oder aus folgenden Gattungen grampositiver Bakterien: Streptococcus und Rothia ausgewählt sind.

4. Gemisch gemäß einem der vorstehenden Ansprüche dadurch gekennzeichnet, daß es äußere Membranen von Klebsiella pneumoniae, Haemophilus influenzae, Branhamella catarrhalis und Zellwände von Streptococcus pneumoniae enthält.

5. Verfahren zur Herstellung des Gemisches gemäß Anspruch 1 - 4 dadurch gekennzeichnet, daß man die äußeren Membranen gramnegativer Bakterien durch Ablösen der Membran vom Sphäroplasten und anschließende Abtrennung der Membran vom Sphäroplasten gewinnt und / oder daß man die Zellwände grampositiver Bakterien durch mechanischen Aufschluß der Bakterien und Abtrennung der Zellwände von den restlichen Zellbestandteilen durch Zentrifugation gewinnt, daß man sie reinigt und daß man die gereinigten äußeren Membranen gramnegativer Bakterien und / oder die gereinigten Zellwände grampositiver Bakterien miteinander mischt.

6. Verfahren gemäß Anspruch 5 dadurch gekennzeichnet, daß man im Falle der Gewinnung von äußeren Membranen gramnegativer Bakterien nach Ablösung der äußeren Membran vom Sphäroplasten das resultierende Gemisch einer Cross-flow-Filtration unterwirft und dabei die äußeren Membranen gewinnt..

7. Arzneimittel für Mensch und Tier bestehend aus oder enthaltend ein Gemisch gemäß einem der Ansprüche 1 bis 4.
